# EUROPÄISCHE PATENTSCHRIFT

(11) **EP 4 295 883 B1**
(45) Veröffentlichungstag und Bekanntmachung des Hinweises auf die Patenterteilung: **15.10.2025**
(21) Anmeldenummer: 23178605.4
(22) Anmeldetag: 12.06.2023
(51) Int. Cl.: A61M 16/00

(54) **VORRICHTUNG UND SYSTEM ZUR ATEMTHERAPIE**
APPARATUS AND SYSTEM FOR RESPIRATORY THERAPY
APPAREIL ET SYSTÈME DE THÉRAPIE RESPIRATOIRE

(30) Priorität: 21.06.2022 DE 102022115391
(43) Veröffentlichungstag der Anmeldung: 27.12.2023
(73) Patentinhaber: Löwenstein Medical Technology S.A., 2557 Luxembourg (LU)
(72) Erfinder: Göbel, Christof, 22457 Hamburg (DE); Schattner, Jan, 25421 Pinneberg (DE)
(74) Vertreter: Löwenstein Medical IP

(56) Entgegenhaltungen:
- EP-B1- 3 212 261
- US-A1- 2013 269 699
- US-A1- 2014 007 877
- US-A1- 2019 151 581

## Beschreibung

Die Erfindung betrifft eine Atemtherapie bei Patienten mit gestörtem oder schwachem Husten, bei denen ein Abhusten von Sekret in den Atemwegen - z.B. basierend auf einem Mangel an muskulärer Kraft - erschwert oder nicht möglich ist (insuffizienter Hustenstoß).

Atemwegsekret hat thixotrope Eigenschaften, das heißt, Scherung durch vorbeiströmendes Gas im Atemweg verursacht unterhalb eines gewissen Grades zunächst keine Verflüssigung oder Bewegung von Sekret. Erst durch bei Überschreitung eines Schwellwertes der Scherung verändert sich die Viskosität des Sekrets und es wird in Bewegung gesetzt. Die Erreichung dieses Grades der Scherspannung durch das vorbeiströmende (exspiratorische) Gas setzt einen gewissen Flow (Peak Flow, Peak Cough Flow) während des Hustens voraus. Dies wiederum ist an bestimmte Voraussetzungen geknüpft und erfordert ohne maschinelle Unterstützung ein Mindestmaß an muskulärer Kraft.

Der Vorgang eines suffizienten Hustens kann in 3 Phasen eingeteilt werden. Auf die erste Phase einer möglichst tiefen Insufflation und Füllung der Lunge (mit geöffneter Glottis) folgen das Schließen der Glottis mit unmittelbar folgender Kompression und Druckaufbau bei geschlossener Glottis und schließlich der Hustenstoß bei wieder geöffneter Glottis, bei dem ein möglichst großer Peak Flow (Peak Cough Flow, PCF) erzeugt werden soll.

Eine Atemtherapie kann für Patienten bei Vorliegen einer Husteninsuffizienz bzw. einer mangelnden muskulären Kraft notwendig sein, um die Entfernung Sekret aus den Atemwegen zu unterstützen. Eine solche Therapie kann eine mechanische (pneumatische) Insufflation/Exsufflation durch ein Atemtherapiegerät umfassen. In der Exsufflations-Phase wird dabei die Energie für die Erzeugung des Luftstroms während des Hustenstoßes durch das Atemtherapiegerät aufgebracht oder der natürliche Hustenstoß zumindest unterstützt.

Für einen suffizienten - mechanisch unterstützten - Husten gehört neben der Aufwendung der notwendigen Energie durch das Atemtherapiegerät auch eine gute Synchronisation der verschiedenen beschriebenen Phasen zwischen Atemtherapiegerät und Patient. Würde beispielsweise der Glottisschluss durch den Patienten vor dem Husten zu spät erfolgen, nachdem das Therapiegerät bereits in die Exsufflationsphase umgeschaltet hat, würde exspiratorischer Fluss ohne Husten (ohne den erforderlichen Peak Flow) generiert werden: der Husten wäre dann nicht effektiv. Würde der Glottisschluss durch den Patienten andererseits verfrüht - also vor Abschluss der Insufflationsphase - erfolgen, wäre ggf. die Lunge noch unzureichend gefüllt und der nachfolgende Husten wäre wiederum nicht optimal und ggf. auch unzureichend. Fallen darüber hinaus die geräteseitige mechanische Unterstützung und der vom Patienten aufgebaute Muskeldruck zeitlich nicht zusammen, wird ebenfalls nicht der individuell bestmögliche Hustenstoß und Peak Cough Flow erzielt. Aus diesen genannten Gründen ist die Synchronisation zwischen dem Patienten und dem Atemtherapiegerät von großer Bedeutung für eine suffiziente mechanische Hustentherapie.

In- und Exsufflationszeiten können mittels einstellbaren Zeitvorgaben erfolgen, wobei dem einstellenden Arzt die Verantwortung obliegt, geeignete Zeiten zu wählen und am Atemtherapiegerät einzustellen.

Die Wahl der Insufflationszeit kann alternativ auch automatisch und auf der Basis von respiratorischen Messwerten bestimmt werden. Gemäß diesem Verfahren wird auf der Basis der Messwerte und von Detektorsignalen auf patientenseitige Veränderungen wie dem Grad der (zunehmenden) Lungenfüllung und/oder die Durchgängigkeit der oberen Atemwege reagiert. Messtechnisch feststellbare und erfindungsgemäß relevante Grenzzustände können beispielsweise eine mindestens weitgehend gefüllte Lunge oder ein weitgehender oder vollständiger Glottisschluss (am Ende der Insufflationsphase) sein.

Zur Optimierung dieser Synchronisation können nach dem Stand der Technik optische oder akustische Signale eingesetzt werden, die den Patienten auf einen Wechsel zwischen diesen Phasen hinweisen. Diese geräteseitig generierten Signale können auf der Basis von geräteseitig hinterlegten oder auch anwenderseitig über eine Nutzerschnittstelle einstellbare Zeiten erfolgen.

In einer klinischen Anwendung kann ein Arzt den mechanisch unterstützten Hustenstoß durch manuelles Komprimieren des Magenbereichs des Patienten während der Exsufflation weiter verbessern. Diese Kompression muss dabei im richtigen Moment, nämlich synchronisiert mit der Ausatmung oder dem Hustenstoß des Patienten erfolgen.

Die EP 2 651 477 B1 offenbart ein Atemtherapiegerät, welches dem Arzt den richtigen Moment für die unterstützende Kompression über ein Tonsignal oder ein Bild- oder ein Licht-Signal angibt.

Die EP 3 212 264 B1 offenbart eine Vorrichtung zur Atmungstherapie eines Patienten mit Husten. Die Vorrichtung gibt dem Patienten einen Hinweis in Form eines Druckanstiegs, um auf bevorstehende Hustenmanöver hinzuweisen.

Die US 2014/0007877 A1 offenbart eine Vorrichtung zur Atmungstherapie eines Patienten während einer Geburt. Die Vorrichtung gibt dem Patienten Hinweise unterschiedliche Atmungsaufgaben zu erfüllen.

Die US 2019/0151581 A1 offenbart eine Vorrichtung zur Atmungstherapie eines Patienten mit Husten. Die Vorrichtung generiert dabei eine Oszillation im Atemgas, um Sekrekt in der Lunge zu lösen und ein Abhusten somit zu erleichtern.

Die US 2013/0269699 A1 offenbart eine Vorrichtung zur Atmungstherapie eines Patienten mit einer Atemgasquelle zur Vorgabe unterschiedlicher Atemgasparameter, mit zumindest einer Steuereinheit und mit einer Signaleinheit zur Ausgabe von zumindest einem Signal, wobei das Signal ein Zeitsignal ist, welches der Signalisierung sich ändernder Atemgasparameter dient und für den Patienten sensorisch wahrnehmbar ist.

Die Nachteile sind, dass das Umfeld durch akustische Signale gestört werden kann oder dass die Wahrnehmung akustischer Signale durch ein lautes Umfeld erschwert sein kann.

Hinsichtlich der Wahrnehmung optischer Signale aus dem Umfeld kann auf das Therapiegerät einfallendes Licht (direktes Sonnenlicht) störend einwirken, und die Wahrnehmung dieser bereitgestellten optischen Signale beeinträchtigen. Ein wesentlicher Nachteil ist jedoch, dass ein Arzt oder Helfer benötigt wird.

Ferner muss der Patient gemäß dem Stand der Technik seine akustischen oder optischen Sinne einsetzen, während seine Konzentration und Fokussierung vorzugsweise auf das intensive mechanische Hustenmanöver - also quasi nach innen - gerichtet ist.

Ein weiterer Aspekt in typischen Anwendungsszenarien ist, dass der Einsatz medizinischer Geräte ohnehin oft von - als störend empfundenen - akustischen Reizen begleitet wird, beispielsweise durch die Eigengeräusche der Geräte oder auch durch - teils unnötige - Alarme der Geräte. Dementsprechend ist es die Aufgabe der vorliegenden Erfindung eine Vorrichtung zur Atmungstherapie zur Verfügung zu stellen, welches die Mängel überwindet.

Ein wesentlicher Vorteil der vorliegenden Erfindung ist, dass der Patient die Signalisierung sich ändernder Atemgasparameter auf dem Luftweg und damit sehr unmittelbar und gut spürbar erhält und er somit in der Lage ist eine optimale Synchronisierung des eigenen Hustenstoßes mit dem Atemtherapiegerät zu erreichen.

Ein weiterer Vorteil der vorliegenden Erfindung ist, dass die erfindungsgemäßen Signale nicht andere Sinne wie Auge oder Ohr des Patienten ansprechen, während er seine ganze Aufmerksamkeit auf den Vorgang der Lungenfüllung und Vorbereitung auf den Hustenstoß gerichtet ist.

Ein besonderer Vorteil der vorliegenden Erfindung ist, dass die erfindungsgemäßen Signale mit seinem Atemapparat wahrnehmen kann.

Erfindungsgemäß umfasst der Atemapparat dabei zumindest die Lunge, die oberen und unteren Atemwege und auch Bereiche des Gesichts, die mit Atemgas in Kontakt stehen können.

Ein weiterer Vorteil der vorliegenden Erfindung ist, dass die erfindungsgemäßen Signale für seh- und/oder hörgeschädigte Personen, die nicht oder nur eingeschränkt akustische oder optische Reize wahrnehmen können besonders geeignet ist.

Diese Aufgabe wird erfindungsgemäß gelöst durch Bereitstellen einer Vorrichtung zur Atmungstherapie nach Anspruch 1.

Die Erfindung betrifft eine Vorrichtung zur Atmungstherapie eines Patienten mit einer Atemgasquelle zur Vorgabe unterschiedlicher Atemgasparameter, mit zumindest einer Steuereinheit und mit einer Signaleinheit zur Ausgabe von zumindest einem Signal. Das Signal dient der Signalisierung sich ändernder Atemgasparameter und ist für den Patienten sensorisch wahrnehmbar.

Die Vorrichtung kennzeichnet, dass das Signal pneumatisch - also über die Luftstrecke (Vorrichtung, Schlauchsystem; Patienteninterface) - an den Atemweg des Patienten übertragen wird.

In manchen Ausführungsformen kennzeichnet die Vorrichtung, dass das Signal eine Modulation des vorgegebenen Atemgases, hinsichtlich Druck und/oder Fluss und/oder Volumen, beinhaltet.

In manchen Ausführungsformen kennzeichnet die Vorrichtung, dass das Signal bevorzugt für den Patienten über seine Mechanorezeptoren sensorisch wahrnehmbar ist.

In manchen Ausführungsformen kennzeichnet die Vorrichtung, dass die Signaleinheit eingerichtet und ausgebildet ist, solche Signale zu erzeugen, die für den Patienten über seine Mechanorezeptoren wahrnehmbar sind.

In manchen Ausführungsformen kennzeichnet die Vorrichtung, dass die Signaleinheit eingerichtet und ausgebildet ist, solche Signale zu erzeugen, die für den Patienten über seine Mechanorezeptoren und akustisch wahrnehmbar sind.

In manchen Ausführungsformen kennzeichnet die Vorrichtung, dass das Signal nicht optisch wahrnehmbar ist und kein Lichtsignal ist.

Die Vorrichtung kennzeichnet, dass die Signaleinheit zur Erzeugung von pneumatischen Signalen ausgebildet ist und beispielsweise ein Ventil und/oder ein Gebläse aufweist.

In manchen Ausführungsformen kennzeichnet die Vorrichtung, dass die Atemgasquelle ein Gebläse ist, welches auch als Signaleinheit dient.

In manchen Ausführungsformen kennzeichnet die Vorrichtung, dass das Signal von der Atemgasquelle erzeugt wird.

In manchen Ausführungsformen kennzeichnet die Vorrichtung, dass die Signaleinheit von der Steuereinheit gesteuert wird.

In manchen Ausführungsformen kennzeichnet die Vorrichtung, dass der sich ändernde Atemgasparameter der durch die Vorrichtung bereitgestellte Atemgasdruck ist.

Die Vorrichtung kennzeichnet, dass der Atemgasparameter beim Umschalten von der Insufflation in die Hustenphase (Exspiration und/oder Exsufflation) verändert wird.

In manchen Ausführungsformen kennzeichnet die Vorrichtung, dass der sich ändernde Atemgasparameter ein positiver Atemgasdruck ist, der im Anschluss an die Insufflationsphase für die Hustenphase abgebaut (Exspiration) wird oder auf Unterdruck (Exsufflation) umgeschaltet wird.

Die Vorrichtung kennzeichnet, dass die Vorrichtung eine Insufflation mit überlagerter Oszillation durchführt, wobei das Signal eine Oszillation ist, die vor der Umschaltung in die Hustenphase (Exsufflation und/oder Exspiration) verändert wird.

In manchen Ausführungsformen kennzeichnet die Vorrichtung, dass das Signal darin besteht, dass die Oszillation vor dem Umschalten in die Hustenphase abgeschaltet wird.

In manchen Ausführungsformen kennzeichnet die Vorrichtung, dass das Signal darin besteht, dass eine vorhandene Oszillation verstärkt wird.

In manchen Ausführungsformen kennzeichnet die Vorrichtung, dass das Signal eine Modulation von Druck und/oder Flow (pneumatisches Signal) aufweist.

Die Vorrichtung kennzeichnet, dass das Signal eine aufmodulierte Schwingung/Oszillation mit einer festen oder sich ändernden Frequenz und/oder Amplitude ist.

In manchen Ausführungsformen kennzeichnet die Vorrichtung, dass die Schwingung eine Frequenz zwischen einem und 40 Hz aufweist.

In manchen Ausführungsformen kennzeichnet die Vorrichtung, dass die Schwingung bevorzugt eine Frequenz zwischen 2 und 30 Hz hat.

In manchen Ausführungsformen kennzeichnet die Vorrichtung, dass die Schwingung wiederum bevorzugt eine Frequenz zwischen 3 und 15 Hz hat

In manchen Ausführungsformen kennzeichnet die Vorrichtung, dass die Amplitude der Schwingung (Peak to Peak) bei 0,2 bis 50 hPa liegt.

In manchen Ausführungsformen kennzeichnet die Vorrichtung, dass die Amplitude der Schwingung (Peak to Peak) bevorzugt bei 0,5 bis 30 hPa liegt.

In manchen Ausführungsformen kennzeichnet die Vorrichtung, dass die Amplitude der Schwingung (Peak to Peak) wiederum bevorzugt bei 1 bis 20 hPa liegt.

In manchen Ausführungsformen kennzeichnet die Vorrichtung, dass die Frequenz und/oder Amplitude sich ansteigend ändern oder sich abfallend ändern.

In manchen Ausführungsformen kennzeichnet die Vorrichtung, dass die Frequenz und/oder Amplitude sich dynamisch ändern.

In manchen Ausführungsformen kennzeichnet die Vorrichtung, dass das Signal ein kurzer positiver oder negativer Druck- und/oder Flowimpuls auf der Luftsäule ist.

In manchen Ausführungsformen kennzeichnet die Vorrichtung, dass die Vorrichtung einen AtemgasDruck ausgehend von einem bestimmten Druck gegen Ende der Insufflationsphase kurzfristig anhebt und anschließend absenkt zurück auf das vorherige Niveau (Plateau).

In manchen Ausführungsformen kennzeichnet die Vorrichtung, dass der Druck gegen Ende der Insufflationsphase zumindest einmal auf den dann höchsten Druck angehoben wird.

In manchen Ausführungsformen kennzeichnet die Vorrichtung, dass der Druck kurzfristig absinkt und dann wieder auf das vorherige Niveau (Plateau) ansteigt.

In manchen Ausführungsformen kennzeichnet die Vorrichtung, dass der Druck zum Ende der Insufflationsphase wieder geringfügig abfällt.

In manchen Ausführungsformen kennzeichnet die Vorrichtung, dass eine (temporäre) Druckanpassung im Bereich von 0,2 bis 8 hPa liegt.

In manchen Ausführungsformen kennzeichnet die Vorrichtung, dass eine (temporäre) Druckanpassung im Bereich von 0,5 bis 5 hPa liegt.

In manchen Ausführungsformen kennzeichnet die Vorrichtung, dass sich das Signal zwischen dem Einsetzen des Signals und dem Moment der Umschaltung verändert.

In manchen Ausführungsformen kennzeichnet die Vorrichtung, dass die Vorrichtung zudem einen Generator zur Erzeugung eines Detektorsignals und einen Sensor zur Ermittlung einer Veränderung des Detektorsignals aufweist, wobei das Detektorsignal geeignet ist, Veränderungen der Durchgängigkeit der Atemwege (Verschluss der Glottis) und/oder eine zumindest fortgeschrittene oder vollständige Füllung der Lunge zu detektieren und der Sensor diese Veränderung des Detektorsignals ermittelt.

In manchen Ausführungsformen kennzeichnet die Vorrichtung, dass eine automatische Umschaltung in die Hustenphase erfolgt, wenn basierend auf dem Detektorsignal eine Veränderung der Durchgängigkeit der Atemwege (Verschluss der Glottis) oder eine zumindest fortgeschrittene oder vollständige Füllung der Lunge ermittelt wird.

In manchen Ausführungsformen kennzeichnet die Vorrichtung, dass das Detektorsignal eine oszillatorische Anregung und/oder Antwort ist und dass eine Änderung der Atemwegs-Impedanz über eine Veränderung des Detektorsignals ermittelt wird.

In manchen Ausführungsformen kennzeichnet die Vorrichtung, dass bei plötzlicher Änderung (Anstieg) der Atemwegs-Impedanz messtechnisch auf einen Verschluss der Glottis geschlossen wird.

In manchen Ausführungsformen kennzeichnet die Vorrichtung, dass die automatische Umschaltung auf einem oszillatorischen Druck-, Fluss- und/oder Volumensignal basiert.

In manchen Ausführungsformen kennzeichnet die Vorrichtung, dass die Umschaltung auch vor Ablauf einer hinterlegten oder eingestellten Zeitdauer stattfindet, wenn der Glottisschluss messtechnisch durch ein Detektorsignal festgestellt ist.

In manchen Ausführungsformen kennzeichnet die Vorrichtung, dass die voreingestellte Zeitdauer dann zu einer max. Dauer wird, die basierend auf einem Detektorsignal auch verkürzt werden kann.

In manchen Ausführungsformen kennzeichnet die Vorrichtung, dass basierend auf dem oszillatorischen Drucksignal zwischen offener und geschlossener Glottis unterschieden werden kann.

In manchen Ausführungsformen kennzeichnet die Vorrichtung, dass basierend auf dem oszillatorischen Drucksignal auf den Füllungsgrad der Lunge geschlossen werden kann
In manchen Ausführungsformen kennzeichnet die Vorrichtung, dass die Oszillation während der Insufflation stattfindet, wobei die Oszillation gleichzeitig als Basis für das Detektorsignal verwendet wird und eine Umschaltung dann erfolgt, wenn basierend auf dem Detektorsignal auf einen Verschluss der Glottis und/oder auf eine zumindest weitgehend vollständig gefüllte Lunge vor Ablauf der eingestellten Insufflationszeit geschlossen werden kann

In manchen Ausführungsformen kennzeichnet die Vorrichtung, dass die Oszillation als Basis für das Detektorsignal für den Glottisschluss im Verlauf der Insufflation hinzugeschaltet wird, wobei die Insufflation durch einen erkannten Glottisschluss oder mit Ablauf der eingestellten maximalen Insufflationszeit beendet wird.

In manchen Ausführungsformen kennzeichnet die Vorrichtung, dass das pneumatische Signal für eine feste und/oder einstellbare Zeitdauer generiert wird

In manchen Ausführungsformen kennzeichnet die Vorrichtung, dass das pneumatische Signal ab einer festen und/oder einstellbaren Zeit vor der Umschaltung in die Hustenphase generiert wird.

In manchen Ausführungsformen kennzeichnet die Vorrichtung, dass die Zeitdauer der Generierung des Signals zwischen 0,1 Sekunden und 2 Sekunden liegt.

In manchen Ausführungsformen kennzeichnet die Vorrichtung, dass die Zeitdauer der Generierung des Signals zwischen 0,5 Sekunden und 1,5 Sekunden liegt.

In manchen Ausführungsformen kennzeichnet die Vorrichtung, dass die Zeitdauer der Generierung des Signals zwischen 0,8 Sekunden und 1,2 Sekunden liegt.

In manchen Ausführungsformen kennzeichnet die Vorrichtung, dass die Zeit vor dem Umschalten von Insufflation zu Exsufflation und/oder Exspiration, zu der mit der Generierung des Signals begonnen wird, zwischen 0,1 Sekunden und 2 Sekunden liegt.

In manchen Ausführungsformen kennzeichnet die Vorrichtung, dass die Zeit vor dem Umschalten von Insufflation zu Exsufflation und/oder Exspiration, zu der mit der Generierung des Signals begonnen wird, zwischen 0,5 Sekunden und 1,5 Sekunden liegt.

In manchen Ausführungsformen kennzeichnet die Vorrichtung, dass die Zeit vor dem Umschalten von Insufflation zu Exsufflation und/oder Exspiration, zu der mit der Generierung des Signals begonnen wird, zwischen 0,8 Sekunden und 1,2 Sekunden liegt.

In manchen Ausführungsformen kennzeichnet die Vorrichtung, dass das pneumatische Signal über eine einstellbare Zeit vor der Umschaltung in die Exsufflation generiert wird.

In manchen Ausführungsformen kennzeichnet die Vorrichtung, dass das pneumatische Signal aufgrund von Druck und Flowwerten generiert wird.

In manchen Ausführungsformen kennzeichnet die Vorrichtung, dass Druck und/oder Flow während eines Manövers gemessen werden.

In manchen Ausführungsformen kennzeichnet die Vorrichtung, dass das pneumatische Signal auf der Basis des Detektorsignales während der Insufflationsphase generiert wird.

In manchen Ausführungsformen kennzeichnet die Vorrichtung, dass, wenn der Flow nahe 0 l/min (also die Patientenlunge voll gefüllt) ist, das pneumatische Signal generiert wird.

In manchen Ausführungsformen kennzeichnet die Vorrichtung, dass das Signal durch ein in die Vorrichtung integriertes Ventil generiert wird.

In manchen Ausführungsformen kennzeichnet die Vorrichtung, dass das Signal durch einen in die Vorrichtung integrierten Aktor generiert wird, der nach dem Verdrängerprinzip arbeitet.

In manchen Ausführungsformen kennzeichnet die Vorrichtung, dass der nach dem Verdrängerprinzip arbeitende Aktor ein Kolben ist.

In manchen Ausführungsformen kennzeichnet die Vorrichtung, dass eine Oszillation mittels einer oszillatorischen Druck-/Flowpumpe generiert wird.

In manchen Ausführungsformen kennzeichnet die Vorrichtung, dass die Oszillation und/oder der Drucksprung mittels eines in die Vorrichtung integrierten Ventils generiert.

In manchen Ausführungsformen kennzeichnet die Vorrichtung, dass die Flow- und/oder Druckverminderung der Oszillation durch veränderliche Leckagen generiert wird.

In manchen Ausführungsformen kennzeichnet die Vorrichtung, dass die Flow- und/oder Druckverminderung der Oszillation durch wechselseitige Verbindung der Atemwege mit der Druck- und Saugseite mindestens einer Flowquelle generiert wird.

In manchen Ausführungsformen kennzeichnet die Vorrichtung, dass die Flow- oder Druckquelle ein Gebläse ist.

In manchen Ausführungsformen kennzeichnet die Vorrichtung, dass die Oszillation und/oder der Drucksprung durch Volumenverschiebung(en) generiert wird.

In manchen Ausführungsformen kennzeichnet die Vorrichtung, dass das Signal durch Variation und/oder Anpassung der Drehzahl des mindestens einen in die Vorrichtung integrierten Gebläses generiert wird.

In manchen Ausführungsformen kennzeichnet die Vorrichtung, dass die Vorrichtung gegen Ende der Insufflationsphase den Druck vorübergehend während einer Plateauzeit auf einem Niveau hält und die Signaleinheit die Ausgabe eines Signales vor dem Umschalten auf einen geringeren Druck initiiert, sodass der Patient die Glottis schließen kann bevor der Druck abgebaut wird und der Hustenstoß beginnt.

Die Erfindung betrifft auch ein System zur Atmungstherapie eines Patienten umfassend die erfinderische Vorrichtung, wobei das System zudem ein Patienteninterface und einen Atemgasschlauch umfasst. Es ist darauf hinzuweisen, dass die in den Ansprüchen und der Beschreibung einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Die Vorrichtung zur Atmungstherapie eines Patienten umfasst zumindest eine Atemgasquelle zur Vorgabe unterschiedlicher Atemgasparameter, zumindest eine Steuereinheit und zumindest eine Signaleinheit zur Ausgabe von zumindest einem Signal. Das Signal ist sensorisch für den Patienten wahrnehmbar und dient der Signalisierung sich ändernder Atemgasparameter. Sich ändernde Atemgasparameter sind im Kontext der Erfindung insbesondere von der Vorrichtung verursachte Änderungen der Atemgasparameter. In manchen Ausführungsformen wird das Signal zumindest pneumatisch, also über die Luftstrecke auf den Atemweg des Patienten übertragen. Zum Beispiel umfasst die Luftstrecke zumindest die Vorrichtung, ein mit der Vorrichtung gekoppeltes Schlauchsystem und ein mit dem Schlauchsystem gekoppeltes Patienteninterface. In manchen Ausführungsformen besteht das Signal in einer Modulation des Druckes und/oder Flusses zum Patienten.

In manchen Ausführungsformen dient das Signal der Signalisierung sich ändernder Atemgasparameter in Form des bevorstehenden Umschaltens von der mechanischen Insufflation zur Exspiration und/oder der Exsufflation im Zuge einer Hustentherapie und/oder Hustenunterstützung. Die Exspiration und/oder Exsufflation stellt eine Hustenphase dar. Das Signal signalisiert also ein Umschalten in die Hustenphase. In manchen Ausführungsformen folgt während einer Hustentherapie auf die Insufflation ein plötzliches Abschalten des positiven Insufflationsdrucks, wodurch eine Exspiration forciert und gegebenenfalls auch ein Hustenstoß des Patienten bewirkt wird. Es wird also von der Insufflation auf die Exsufflation und/oder die Exspiration umgeschaltet. In manchen Ausführungsformen folgt auf die Insufflation eine Exsufflation, welche eine höhere Druckdifferenz zur Insufflation erzeugt und damit auch eine höhere Energie für die Hustenreaktion bewirkt. In manchen Ausführungsformen weist das Signal den Patienten daraufhin, dass er manuell auf die Exspiration und/oder Exsufflation umschalten kann bzw. sollte.

In manchen Ausführungsformen ist das Signal unabhängig davon, ob die Hustenphase durch ein, auf eine Insufflation folgendes, Umschalten in eine Exspiration oder Exsufflation beginnt. Mit einem Umschalten in die Hustenphase ist in manchen Ausführungsformen insbesondere ein Umschalten zur Exspiration und/oder Exsufflation gemeint.

Es kann in manchen Ausführungsformen vorgesehen sein, dass die Insufflation mit einer überlagerten Oszillation durchgeführt wird, wobei das Signal darin besteht, dass die Oszillation vor der Umschaltung der Insufflation auf eine Exspiration und/oder Exsufflation zu verändern und/oder abzuschalten. Eine Veränderung der Oszillation kann beispielsweise über eine Verstärkung oder Abschwächung der Oszillation erfolgen. Beispielsweise wird die Amplitude und/oder die Frequenz der Oszillation erhöht oder verringert.

Wird die Insufflation ohne überlagerte Oszillation durchgeführt, ist vorgesehen, dass das Signal darin besteht eine spezifische Modulation vor der Umschaltung in die Hustenphase mit Exsufflation bzw. Exspiration durchzuführen.

In manchen Ausführungsformen ist vorgesehen, dass besteht das Signal in einer Modulation von Druck und/oder Fluss, beispielsweise als pneumatisches Signal. Es kann etwa vorgesehen sein, dass das Signal eine Schwingung und/oder Oszillation des Druckes und/oder des Flusses ist. Dabei hat die Schwingung/Oszillation beispielsweise eine Frequenz zwischen 1 Hz und 40 Hz, bevorzugt 2 Hz und 30 Hz, mehr bevorzugt zwischen 3 Hz und 15 Hz. Die Peak-to-Peak-Amplitude der Schwingung/Oszillation liegt beispielsweise bei 0,2 hPa bis 50 hPa, beispielsweise bei 0,5 hPa bis 30 hPa. In manchen Ausführungsformen liegt die Amplitude bei 1 hPa bis 20 hPa.

Alternativ oder ergänzend ist das Signal ein kurzer positiver oder negativer Druck- und/oder Flussimpuls auf der Luftsäule, beispielsweise in der Druck-Plateauphase, wobei der Impuls relativ zum Plateaudruck und/oder -fluss zu verstehen ist. Beispielsweise steigt der Druck zur Signalisierung kurzfristig an und sinkt dann auf das vorherige Niveau, z.B. den Plateaudruck, zurück. In manchen Ausführungsformen kann dabei vorgesehen sein, dass unmittelbar nach dem Absinken auf den Plateaudruck die Umschaltung zur Exsufflation und/oder Exspiration erfolgt. Alternativ kann vorgesehen sein, dass nach dem Absinken auf den Plateaudruck eine kurze Zeit der Plateaudruck aufrechterhalten wird bevor die Umschaltung erfolgt. In manchen Ausführungsformen ist alternativ oder ergänzend vorgesehen, dass für die Signalisierung der Druck zum Ende der Plateauphase auf einen dann höchsten Druck während der Insufflation ansteigt und die Umschaltung dann bei dem höchsten Druck erfolgt. Es kann dabei vorgesehen sein, dass unmittelbar nach Erreichen des dann höchsten Drucks die Umschaltung erfolgt. Alternativ kann vorgesehen sein, dass der dann höchste Druck für eine bestimmte Zeitdauer gehalten wird bevor die Umschaltung erfolgt.

In manchen Ausführungsformen ist vorgesehen, dass das Signal darin besteht, dass der Druck während der Plateauphase kurzeitig absinkt und dann wieder auf das Plateauniveau ansteigt, bevor die Umschaltung erfolgt. Dabei kann vorgesehen sein, dass unmittelbar nach dem Wiederanstieg auf das Plateauniveau die Umschaltung erfolgt oder das Plateauniveau noch eine Zeit lang gehalten wird bevor die Umschaltung erfolgt. In manchen Ausführungsformen kann vorgesehen sein, dass der Druck zur Signalisierung vor der Umschaltung abgesenkt wird und ohne Wiederanstieg die Umschaltung erfolgt.

Die Höhe der (temporären) Druckanpassung - Anstieg oder Absenkung - liegt bei 0,2 hPa bis 8 hPa, bevorzugt bei 0,5 hPa bis 5 hPa.

In manchen Ausführungsformen kann das Signal auch zwischen dem Einsetzen des Signals und der Umschaltung verändern. Beispielsweise kann sich die Druckanpassung während des Signals vergrößern, also ansteigend und/oder vermindernd verlaufen.

In manchen Ausführungsformen ist die Vorrichtung dazu eingerichtet eine automatische Umschaltung in die Hustenphase, also Exspiration und/oder Exsufflation, durchzuführen. Die automatische Umschaltung kann beispielsweise auf einem oszillatorischen Druck-, Fluss- und/oder Volumensignal basieren. Beispielhaft kann vorgesehen sein, dass eine Oszillation des Flusses und/oder Volumens als Anregung durch die Vorrichtung erzeugt wird und die Reaktion der Atemwege auf diese Oszillation, beispielsweise in Form eines Drucksignals, als Antwort gemessen wird. In manchen Ausführungsformen kann vorgesehen sein, dass neben der Antwort auch eine etwaige Phasenverschiebung zwischen Anregung und Antwort erfasst und/oder ausgewertet wird.

In manchen Ausführungsformen kann vorgesehen sein, dass Signal über den Zeitraum aufrechterhalten wird, bis auf Basis der Antwort und/oder eines anderen Detektorsignals eine automatische Umschaltung ausgelöst wird. In manchen Ausführungsformen kann über die Antwort der Atemwege auch eine Unterscheidung zwischen einer gefüllten Lunge mit offener und geschlossener Glottis erfolgen. Beispielsweise kann aus der Antwort eine Änderung der Atemwegs-Impedanz gemessen werden. Aus der Atemwegs-Impedanz kann dann beispielsweise der Zustand der Glottis (z.B. offen/geschlossen, ggf. auch teilweise geschlossen) bestimmt werden. Bei einem beispielsweise plötzlichen Anstieg der Atemwegs-Impedanz kann beispielsweise auf einen Verschluss der Glottis geschlossen werden. Wird der Glottisverschluss etwa messtechnisch festgestellt, kann vorgesehen sein, dass die Umschaltung von Insufflation auf Exspiration bzw. Exsufflation erfolgt. In manchen Ausführungsformen ist eine Zeit hinterlegt und/oder eingestellt, über welche die Insufflation erfolgen soll oder mindestens erfolgen soll. Bei einem festgestellten Glottisverschluss kann die Umschaltung auch vor Ablauf der eingestellten oder hinterlegten Zeit erfolgen. Die eingestellte oder hinterlegte Zeit kann dadurch zum Beispiel auch eine maximale Zeit der Insufflation sein.

In manchen Ausführungsformen umfasst die Vorrichtung eine Einrichtung (Generator) zur Erzeugung eines Detektorsignals sowie einen oder mehrere Sensoren zur Ermittlung von Veränderungen des Detektorsignals. Das Detektorsignal ist beispielsweise dazu geeignet, die Durchgängigkeit und/oder die Veränderung der Durchgängigkeit der Atemwege des Patienten zu detektieren. Eine verminderte Durchgängigkeit der Atemwege kann beispielsweise auf einen Verschluss der Glottis hinweisen. In manchen Ausführungsformen wird ein Verschluss der Glottis als Hinweis darauf genommen, dass der Patient tiefgenug eingeatmet hat und ein Umschalten von Insufflation auf Exsufflation bzw. Exspiration erfolgen kann. Alternativ oder ergänzend kann das Detektorsignal dazu geeignet sein den Füllstand und/oder Veränderungen am Füllstand der Lunge des Patienten zu detektieren.

In manchen Ausführungsformen ist das Detektorsignal ein Fluss-, Volumen- und/oder Drucksignal. In manchen Ausführungsformen wird das Detektorsignal durch eine Oszillation des Drucks und/oder Flusses erzeugt. Es kann beispielhaft vorgesehen sein, dass die Einrichtung zur Erzeugung des Detektorsignals ein Oszillationsventil ist.

In manchen Ausführungsformen kann vorgesehen sein, dass während der gesamten Insufflation eine überlagerte Oszillation stattfindet. Dabei kann diese Oszillation genutzt werden, um über eine Anregung eine Antwort der Atemwege des Patienten, beispielhaft in Form eines oszillatorischen Drucksignals, zu ermitteln, beispielsweise um den Verschluss der Glottis festzustellen. Beispielsweise erfolgt dann eine Umschaltung, wenn ein (ggf. vorzeitiger) Verschluss der Glottis erfolgt.

In manchen Ausführungsformen wird die Oszillation als Signal zur Signalisierung der Umschaltung hinzugeschaltet. Es kann beispielsweise vorgesehen sein, dass eine Zeit eingestellt oder hinterlegt ist, über welche die Insufflation vor dem Umschalten erfolgen soll, wobei das Signal beispielsweise ein Ablauf dieser Zeit und das bevorstehende Umschalten signalisiert. Erfolgt die Signalisierung über eine Oszillation, beispielsweise des Drucks, kann diese Oszillation genutzt werden, um ein oszillatorisches Drucksignal zu ermitteln. Da eine automatische Umschaltung erfolgt, wenn ein Verschluss der Glottis festgestellt wird, wird die hinterlegte oder eingestellte Zeit somit zu einer maximalen Zeit. In manchen Ausführungsformen kann dabei auch eine Mindestzeit eingestellt oder hinterlegt werden, über welchen Zeitraum die Insufflation mindestens erfolgen soll.

In manchen Ausführungsformen ist alternativ oder ergänzend vorgesehen, dass das pneumatische Signal für eine festgelegte Zeitdauer vor der Umschaltung auf die Exsufflation bzw. die Exspiration generiert wird. Der Patient kann sich entsprechend des Signals auf eine Umschaltung vorbereiten. In manchen Ausführungsformen liegt die festgelegte Zeitdauer zwischen 0,1 Sekunden und 2 Sekunden, bevorzugt zwischen 0,5 Sekunden und 1,5 Sekunden. In manchen Ausführungsformen liegt die festgelegte Zeitdauer vor der Umschaltung bevorzugt zwischen 0,8 und 1,2 Sekunden. In manchen Ausführungsformen ist die Zeit ergänzend oder alternativ zwischen 0 Sekunden und 2 Sekunden einstellbar.

In manchen Ausführungsformen kann vorgesehen sein, dass die Intensität des Signals nach einer Mehrzahl an Therapien allmählich geringer wird. Beispielsweise kann vorgesehen sein, so den Patienten an den Umschaltzeitpunkt zu gewöhnen, sodass der Patient immer weniger auf das Signal angewiesen ist und mehr von selbst die bevorstehende Umschaltung erkennt bzw. vorausahnen kann. Eine solche Abschwächung des Signals ist beispielsweise besonders in Verbindung mit einer festgelegten und/oder einstellbaren Zeitdauer der Signalgenerierung vor der Umschaltung sowie einer festgelegten Insufflationszeit nutzbar. In manchen Ausführungsformen kann auch vorgesehen sein, dass die Synchronität zwischen Patienten und Vorrichtung bzw. Therapie ausgewertet wird und bei einer - nach Abschwächung des Signals - zunehmenden Abweichung das Signal wieder verstärkt wird.

Ergänzend oder alternativ ist in manchen Ausführungsformen vorgesehen, dass das Signal anhand von Druck- und/oder Flusswerten generiert wird. Beispielsweise werden der Druck und/oder der Fluss während der Insufflation gemessen. Bei bestimmten Drücken und/oder Flüssen kann dann vorgesehen sein, dass das Signal generiert wird um eine Umschaltung von der Insufflation in die Exspiration bzw. Exsufflation zu signalisieren. Beispielsweise kann vorgesehen sein, dass die während der Beatmung der Druck und/oder Fluss daraufhin ausgewertet wird, ob der Patient tief genug eingeatmet hat bzw. die Insufflation weitestgehend abgeschlossen ist, sodass zeitnah die Umschaltung erfolgt bzw. erfolgen kann. Beispielsweise kann vorgesehen sein, dass das Signal erzeugt bzw. ausgegeben wird, wenn sich der Fluss 0 l/min annähert, die Lunge des Patienten also voll gefüllt ist. In manchen Ausführungsformen ist vorgesehen, dass die Vorrichtung dazu eingerichtet ist, anhand der Fluss- und/oder Druckwerten vorherzusagen, wann die Insufflation abgeschlossen sein wird und eine Umschaltung erfolgt, sodass ab einer festgelegten und/oder einstellbaren Zeit vor der Umschaltung das Signal für eine festgelegte und/oder einstellbare Zeitdauer generiert wird.

Zur Erzeugung des Signals kann beispielhaft vorgesehen sein, dass das Signal, etwa in Form einer Druckanpassung und/oder Oszillation, mittels eines in die Vorrichtung integrierten Ventils generiert wird. Eine Fluss- und/oder Druckverminderung, beispielsweise zur Erzeugung einer Oszillation, kann dabei beispielhaft durch veränderliche Leckagen generiert werden. In manchen Ausführungsformen kann eine Oszillation und/oder Fluss- bzw. Druckanpassung durch eine wechselseitige Verbindung des Atemgaswegs zum Patienten mit der Druck- und Saugseite der Druck- bzw. Flussquelle. Beispielhaft kann vorgesehen sein, dass die Druck- bzw. Fluss ein Gebläse ist.

In manchen Ausführungsformen wird das Signal in Form einer Oszillation und/oder Druckanpassung über Volumenverschiebungen erzeugt. Beispielhaft kann das Signal durch eine Variation und/oder Anpassung der Drehzahl eines Gebläses, beispielsweise der Druck- bzw. Flussquelle, generiert werden. Alternativ können die Volumenverschiebungen auch über ein Verdrängungsprinzip, etwa durch einen Kolben, generiert werden. In manchen Ausführungsformen wird eine Oszillation über eine oszillatorische Druck- und/oder Flusspumpe generiert.

In manchen Ausführungsformen wird das pneumatische Signal durch ein akustisches Signal begleitet. In manchen Ausführungsformen entsteht das akustische Signal durch die Generierung des pneumatischen Signals. In manchen Ausführungsformen kann vorgesehen sein, dass das pneumatische Signal durch ein zusätzlich generiertes akustisches Signal unterstützt wird. Alternativ oder ergänzend kann vorgesehen sein, dass das durch die Generierung des pneumatischen Signals entstehende akustische Signal zusätzlich verstärkt wird.

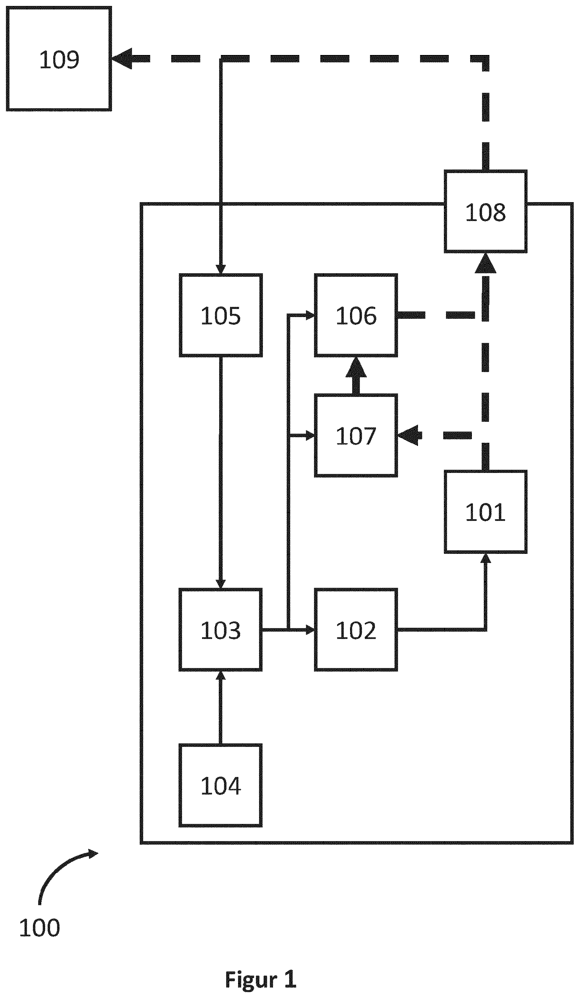

In den Figuren 1 bis 9 wird die Erfindung anhand beispielhafter Ausführungsformen näher beschrieben.

In Figur 1 ist eine beispielhafte Ausführungsform einer Vorrichtung 100 zur Atmungstherapie eines Patienten 109 schematisch dargestellt. Die Vorrichtung 100 umfasst beispielhaft eine Fluss- bzw. Druckquelle in Form eines Gebläses 101, beispielhaft angetrieben durch eine steuerbare Motoreinheit 102. Pneumatisch mit dem Gebläse 101 verbunden sind beispielhaft zumindest ein Ventil 107 und sowie eine Signaleinheit 106 zur Erzeugung eines Signals 204 zur Signalisierung sich ändernder Atemgasparameter. Die Signaleinheit 106 ist beispielhaft gleichzeitig auch ein Oszillationsventil 110 zur Erzeugung einer Oszillation des Druckes und/oder Flusses. Eine pneumatische Verbindung zum Patienten 109 wird über ein Schlauchsystem und Patienteninterface (nicht dargestellt) über den Geräteausgang 108 hergestellt. Über Sensoren 105 werden zum Beispiel Druck- und/oder Flusswerte aufgenommen.

Über die Steuereinheit 103 ist zumindest die Steuerung der Motoreinheit 102 zum Antrieb des Gebläses 101 sowie die Steuerung des Ventils 107 sowie der Signaleinheit 106 realisiert.

Eine beispielhafte Atemtherapie umfasst eine Insufflation 201 des Patienten 109, wobei der Überdruck der Insufflation 201 plötzlich bzw. unmittelbar (<500 ms) durch Umschalten 202 gestoppt wird. Darauffolgend schließt sich eine (forcierte) Exspiration oder eine Exsufflation 203 des Patienten 109 an, im Zuge der Erfindung auch als Hustenphase bezeichnet. Die beispielhafte Vorrichtung 100 ist so eingerichtet, dass mit dem Gebläse 101 eine Insufflation 201 und eine Exsufflation 203 möglich ist. Bei der Exsufflation 203 wir ein Unterdruck (relativ zum Umgebungsdruck) erzeugt. Eine Umschaltung 202 von Insufflation 201 zu Exsufflation 203 ist beispielhaft durch das Ventil 107 möglich. Durch ein Umschalten des Ventils 107 wird die Saugseite des Gebläses 101 von Umgebungsluft auf Atemgasweg umgeschaltet. Während der Insufflation 201 saugt das Gebläse 101 die Umgebungsluft an und fördert diese als Atemgas in die Richtung des Patienten 109. Nach Umschalten ist die Saugseite des Gebläses 101 mit dem Atemgasweg verbunden; das Gebläse saugt also auf der Seite des Patienten 109 an und fördert in Richtung Umgebungsluft. So entsteht während einer Exsufflation 203 ein Unterdruck auf Seite des Patienten. In manchen Ausführungsformen kann vorgesehen sein, dass die Vorrichtung 100 ergänzend oder alternativ so eingerichtet ist, dass während der beispielhaften Atemtherapie auf die Insufflation 201 keine Exsufflation 203 folgt, sondern die Bereitstellung eines Überdrucks nach der Insufflation 201 abrupt endet und somit eine Exspiration und idealerweise ein Husten des Patienten 109 forciert wird.

In manchen Ausführungsformen kann vorgesehen sein, dass ein Umschalten von Insufflation 201 auf eine Exspiration 203a ohne Schalten des Ventils 107 möglich ist. Beispielhaft wird dazu die Drehzahl des Gebläses 101 plötzlich verringert, um einen Druckabfall zu erzeugen.

Die Änderung von Atemgasparametern durch das Umschalten 202 von Insufflation 201 auf Exspiration 203a und/oder Exsufflation 203 wird erfindungsgemäß durch eine Signal 204 signalisiert bzw. angekündigt. Bevorzugt handelt es sich dabei um ein pneumatisches Signal 204, welches in der beispielhaften Vorrichtung 100 über die Signaleinheit 106 erzeugt wird. Beispielhaft ist die Signaleinheit als Oszillationsventil, welches eine Oszillation des Insufflationsdrucks und/oder -fluss erzeugt, wobei die Oszillation zumindest als Signal 204 dient. In manchen Ausführungsformen ist die Insufflation 201 abseits vom Signal 204 mit einer Oszillation überlagert, generiert durch das Oszillationsventil 110. Ein Signal 204 kann beispielsweise durch das Stoppen der Oszillation oder der Modulation der Oszillation, beispielsweise der Amplitude und/oder Frequenzänderung, generiert werden.

Zur Erzeugung des Signals 204 wird dazu die Signaleinheit 106 entsprechend durch die Steuereinheit 103 angesteuert und mit entsprechenden Steuersignalen angesteuert um beispielsweise Vorgaben zu Beginn, Ende, Amplitude und/oder Frequenz des Signals 204 in Form einer Oszillation umzusetzen.

Besteht das Signal 204 in einer Oszillation, so weist die Schwingung eine Frequenz zwischen 1 Hz und 40 Hz, bevorzugt zwischen 2 Hz und 30 Hz auf. In manchen Ausführungsformen ist die Frequenz der Oszillation bevorzugt zwischen 3 Hz und 15 Hz. Die Amplitude der Oszillation, welche als Signal 204 dient, liegt beispielhaft bei 0,2 hPa bis 50 hPa, bevorzugt bei 0,5 hPa bis 30 hPa. In manchen Ausführungsformen liegt die Amplitude der Oszillation des Signals 204 bei 1 hPa bis 20 hPa. Die Amplitude bezeichnet dabei den Abstand beider Peaks zueinander.

Über die Fluss- und/oder Drucksensoren 105 können verschiedene Atemgasparameter erfasst werden. Beispielsweise kann vorgesehen sein, dass über die Oszillation während der Insufflation - sowohl als Signal 204 als auch abseits vom Signal 204 - bestimmt wird, ob und/oder wann ein Umschalten 202 erfolgen soll. Dabei kann vorgesehen sein, dass eine Mindest- und/oder Maximalzeit zur Insufflation eingestellt und/oder vorgegeben ist. Ist die Mindestinsufflationszeit erreicht kann beispielsweise das Signal generiert werden. Das Umschalten kann dann nach einer eingestellten oder festgelegten Zeitdauer, für welche das Signal 204 generiert wird, erfolgen. Alternativ oder ergänzend kann vorgesehen sein, dass die Umschaltung erfolgt, wenn über die Oszillation festgestellt wird, dass ein Umschalten erfolgen soll. In manchen Ausführungsformen wird die einstellbare oder festgelegte Zeitdauer für das Signal 204 zu einer maximalen Zeitdauer, die Umschaltung erfolgt also spätestens nach Ablauf der Signalzeit. In manchen Ausführungsformen ist alternativ oder ergänzend eine Maximalinsufflationszeit vorgesehen, nach deren Ablauf unabhängig von den Oszillationsmessungen die Umschaltung 202 erfolgt. Beispielsweise kann die maximale Insufflationszeit einer Mindestinsufflationszeit plus einer Signalzeit entsprechen. In manchen Ausführungsformen, insbesondere wenn keine Mindestinsufflationszeit vorgesehen ist, kann vorgesehen sein, dass das Signal 204 entsprechend der Dauer des Signals 204 vor dem Ablauf der maximalen Insufflationszeit erzeugt wird.

Über die Sensoren 105 kann alternativ oder ergänzend festgestellt werden, ob die Lunge des Patienten 109 voll gefüllt ist und ein Umschalten 202 von der Insufflation 201 auf die Exspiration 203a bzw. Exsufflation 203 erfolgen soll. Beispielsweise kann eine Annäherung des Flusses auf 0 l/min auf eine vollgefüllte Lunge hindeuten, sodass die Steuereinheit 103 die Signaleinheit 106 so ansteuert, dass das Signal 204 zur Signalisierung sich ändernder Atemgasparameter - hier die Umschaltung von Insufflation auf Exsufflation 203 bzw. Exspiration 203a - generiert wird.

Über die Benutzerschnittstelle 104 sind beispielhaft Eingaben von Daten, Informationen und/oder Parametern möglich. Unter anderem können Parameter zum Signal 204 eingebbar sein. Beispielsweise können Zeit vor dem Umschalten, Zeitdauer, Frequenz, Amplitude und/oder Intensität des Signals 204 eingestellt werden. Die Zeit ist dabei zum Beispiel eine Zeitdauer des Signals 204 und/oder ab wann vor der Umschaltung 202 von Insufflation 201 auf Exsufflation 203 bzw. Exspiration das Signal 204 generiert wird. Beispielsweise kann festgelegt sein und/oder einstellbar sein, dass das Signal über eine Zeitdauer von 0,1 bis 2 Sekunden generiert wird. Bevorzugt liegt die einstellbare und/oder festgelegte Zeitdauer bei 0,5 Sekunden bis 1,5 Sekunden. In manchen Ausführungsformen liegt die einstellbare und/oder festgelegte Zeitdauer bevorzugt bei 0,8 Sekunden bis 1,2 Sekunden.

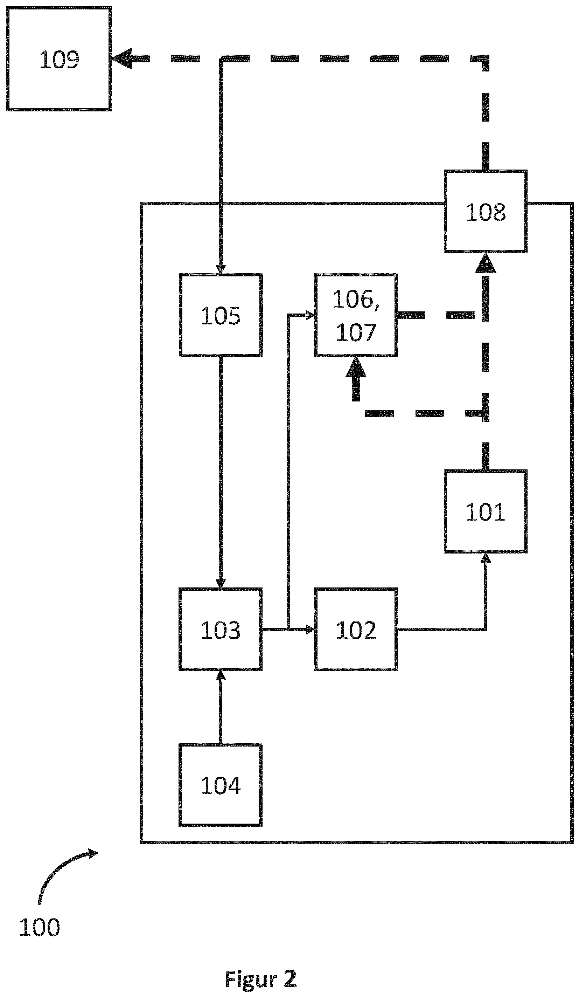

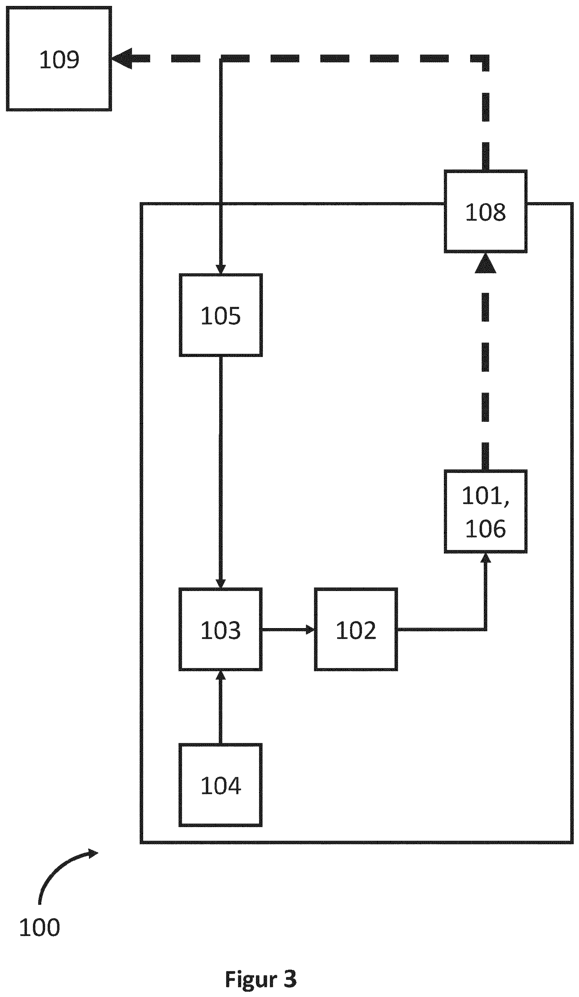

In manchen Ausführungsformen können alternativ oder ergänzend das Gebläse 101 bzw. die Motoreinheit 102 und/oder das Ventil 107 als Signaleinheit 106 ausgebildet sein. Beispielsweise kann vorgesehen sein, dass die Vorrichtung 100 mehrere Signaleinheiten 106 umfasst, welche verschiedene Arten von Signalen 204 erzeugen können. Wie das Gebläse 101 und/oder das Ventil 107 als Signaleinheiten 106 eingesetzt werden können wird beispielhaft näher in den Figuren 2 und 3 erläutert.

Eine weitere beilspielhafte Ausführungsform der Vorrichtung 100 ist in Figur 2 schematisch dargestellt. Die beispielhafte Vorrichtung 100 weist zumindest eine Atemgasquelle in Form eines Gebläses 101, welches von einer durch die Steuereinheit 103 steuerbare Motoreinheit 102 angetrieben wird.

Die Vorrichtung 100 weist ferner ein Ventil 107 auf, welches zwischen einer Insufflation 201 und einer Exsufflation 203 umschalten kann, wie auch in der in Figur 1 beschriebenen Ausführungsform. Dabei wird die Gasführung so geschaltet, dass die Saugseite des Gebläses 101 während der Insufflation 201 mit einem Ansaugbereich für Umgebungsluft verbunden ist. Zur Exsufflation 203 wird das Ventil so umgeschaltet, dass die Saugseite des Gebläses 101 mit dem Atemgasweg zum bzw. vom Patienten 109 verbunden ist und somit einen Unterdruck zum Patienten 109 erzeugt.

Das Ventil 107 ist zudem als Signaleinheit 106 eingerichtet und kann von der Steuereinheit 103 dazu angesteuert werden, ein pneumatisches Signal 204 zu erzeugen, um sich ändernde Atemgasparameter zu signalisieren. Insbesondere kann das Signal 204 ein Umschalten 202 von der Insufflation 201 in die Exsufflation 203 bzw. die Exspiration 203a signalisieren.

Das Signal 204 kann beispielsweise in einer Anpassung des Drucks- und/oder Flusses während der Insufflation 201 sein. Auch kann vorgesehen sein, dass durch gezielte Schaltung des Ventils 107 eine Oszillation als Signal 204 generiert werden kann.

Beispielhaft kann eine Anpassung des Druckes und/oder Flusses zur Erzeugung des Signals 204 dadurch erfolgen. Dabei wird eine festgelegte und/oder einstellbare Zeit vor dem Umschalten der Druck und/oder der Fluss erhöht bzw. verringert. Eine einmalige, kurze Verringerung des Drucks kann beispielsweise durch ein kurzes Umschalten des Ventils 107 erfolgen, sodass für einen kurzen Moment die Saugseite des Gebläses 101 mit dem Atemgasweg vom/zum Patienten 109 verbunden ist und den Druck dort so kurzeitig verringert. In manchen Ausführungsformen kann, beispielsweise für eine längere Zeit, in der ein geringerer Druck als Signal 204 dienen soll, eine Verringerung der Drehzahl des Gebläses 101 vorgesehen sein. Gleichermaßen kann eine Erhöhung des Druckes als Signal 204 durch eine Erhöhung der Drehzahl des Gebläses 101 erreicht werden.

Die Sensoreinheit 105 sowie die Benutzerschnittstelle 104 sind beispielhaft wie in der Ausführungsformen der Figur 1 ausgeführt.

Figur 3 zeigt schematisch eine beispielhafte Ausführungsform der Vorrichtung 100 zur Atmungstherapie eines Patienten 109. Die Vorrichtung 100 weist eine Atemgasquelle in Form eines von einer Motoreinheit 102 angetriebenes Gebläses 101 auf, welches gleichzeitig auch als Signaleinheit 106 ausgebildet ist. Das Gebläse 101 ist beispielhaft dazu eingerichtet während einer Insufflation 201 dem Patienten 109 einen Überdruck bereitzustellen. Durch ein plötzliches Umschalten auf die Exspiration 203a, beispielsweise plötzliches Abbremsen des Gebläses 101, kann beim Patienten 109 eine Exspiration forciert werden und ein Husten ausgelöst werden. Es wird also von der Insufflation 201 in die Hustenphase umgeschaltet. Um unter anderem diese sich ändernden Atemgasparameter zu signalisieren kann durch die Signaleinheit 106 ein pneumatisches Signal erzeugt werden.

Zur Generierung des Signals 204 wird der Motor 102 durch die Steuereinheit 103 so angesteuert, dass das Gebläse 101 Variationen in dem bereitgestellten Druck während der Insufflation 201 erzeugen kann. Beispielhaft ist vorgesehen, dass die Generierung des Signals 204 eine Zeit vor dem Umschalten beginnt und dann für eine Zeitdauer andauert.

Beispielsweise kann durch eine periodische Verringerung und Erhöhung der Drehzahl ein oszillatorisches Signal 204 erzeugt werden. In manchen Ausführungsformen kann vorgesehen sein, dass die gesamte Insufflation 201 mit einer Oszillation überlagert wird, das Signal 204 kann dann etwa darin bestehen, dass die Oszillation moduliert wird, also in Frequenz und/oder Amplitude geändert wird. Auch kann vorgesehen sein, dass das Signal 204 durch ein Stoppen der Oszillation generiert wird.

In manchen Ausführungsformen ist vorgesehen, dass das Signal 204 eine Änderung des Drucks und/oder Flusses ist. Beispielhaft wird zur Erzeugung des Signal 204s die Drehzahl des Gebläses 101 erhöht, was zu einem zumindest zeitweise ansteigenden Druck und/oder Fluss führt und dem Patienten 109 sich ändernde Atemgasparameter, insbesondere das Umschalten von Insufflation 201 auf Exspiration 203a, Signal 204isiert. In manchen Ausführungsformen kann vorgesehen sein, dass die Druck- und/oder Flusserhöhung nicht über den gesamten Zeitraum bis zum Umschalten andauert, sondern nur für einen kürzeren Zeitraum vor dem Umschalten. Alternativ oder ergänzend kann als Signal 204 vorgesehen sein, dass das Signal 204 in einer zumindest zeitweisen Verringerung des Druckes liegt. Beispielsweise wird der Druck kurzzeitig abgesenkt und dann wieder auf das Plateauniveau der Insufflation 201 angehoben, bevor das Umschalten erfolgt.

Die Sensoreinheit 105 sowie die Benutzerschnittstelle 104 sind beispielhaft wie in der Ausführungsformen der Figur 1 ausgeführt.

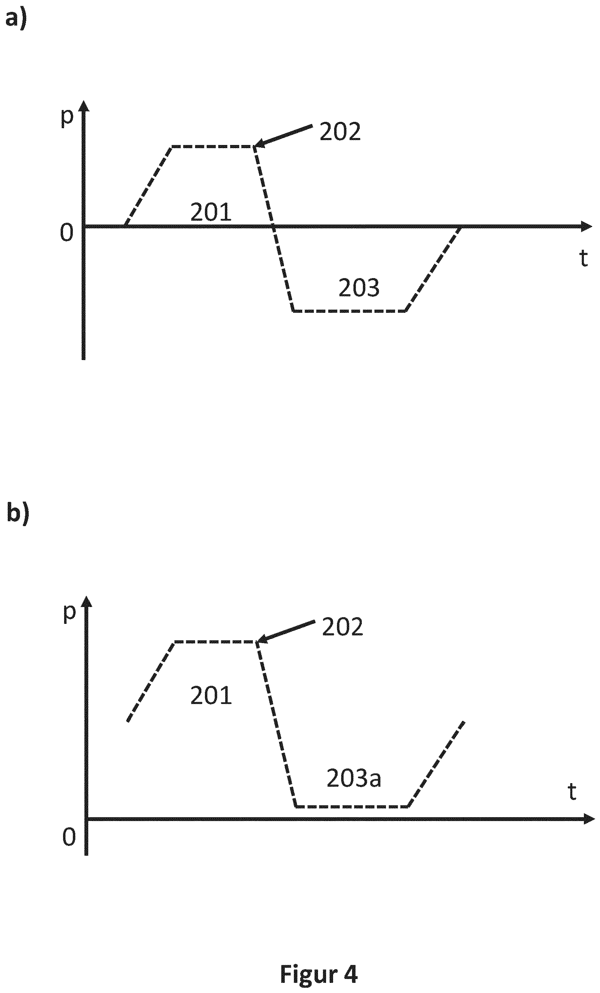

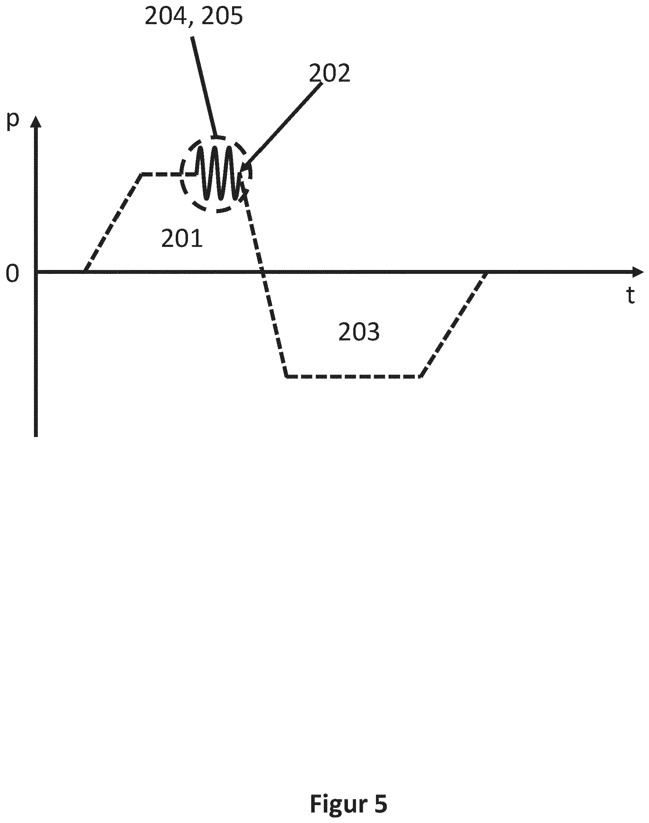

In den Figuren 4 bis 9 sind beispielhaft verschiedene Druckkurven einer Insufflation 201 und Exsufflation 203 bzw. Exspiration 203a schematisch dargestellt. Figur 4a beschreibt beispielhaft einen Druckverlauf der Insufflation 201 und Exsufflation 203 nach dem Stand der Technik, Figur 4b zeigt anstatt einer Exsufflation 203 eine Exspiration 203a. In den Figuren 5 bis 9 sind beispielhaft verschiedene Signale 204 vor dem Umschalten anhand eines Druckverlaufes mit Insufflation 201 und Exsufflation 203 dargestellt. Die Signale 204 können gleichermaßen auch auf Ausführungsformen übertragen werden, bei denen nach dem Umschalten keine Exsufflation 203 erfolgt, sondern ein Umschalten auf eine Exspiration 203a vorgesehen ist. In den dargestellten Diagrammen bezeichnet die x-Achse den Druck p und die y-Achse die Zeit t. Die eingezeichnete Null-Linie bezieht sich dabei relativ zum Umgebungsdruck. Ein Druck p von Null entsprich dabei dem Umgebungsdruck.

Figur 4a zeigt schematisch einen beispielhaften Druckverlauf für eine Insufflation 201 mit anschließender Exsufflation 203 gemäß dem Stand der Technik. Zu Beginn der Insufflation 201 steigt der Druck p an, bis ein Plateauniveau erreicht ist. Dieses Plateau wird eine Zeit gehalten bis die Insufflation 201 beendet ist und ein Umschalten 202 auf die Exsufflation 203 erfolgt. Durch das Umschalten 202 auf die Exsufflation 203 sinkt der Druck schnell ab und forciert den Patienten zu einer Exspiration und/oder einem Husten. Bei der Exsufflation 203 wird am Patienten ein Unterdruck (relativ zum Umgebungsdruck) bereitgestellt bzw. ein Unterdruck in Richtung Atemwege des Patienten 109 appliziert, wodurch die Exspiration bzw. das Husten stärker forciert wird.

Figur 4b zeigt schematisch einen beispielhaften Druckverlauf für eine Insufflation 201 bei der eine Umschaltung 202 in die Exspiration 203a erfolgt. Im Unterschied zur Exsufflation 203 erfolgt hier keine Bereitstellung eines zum Umgebungsdruck relativen Unterdruck, sondern der während der Insufflation 201 bereitgestellte Überdruck wird stark und plötzlich reduziert und nähert sich in manchen Ausführungsformen dem Umgebungsdruck an. Durch die starke Reduzierung des Druckes wird der Patient zu einer Exspiration 203a forciert, gegebenenfalls eihergehend mit einem Husten.

Eine beispielhafte Ausführungsform des Signals 204 ist in Figur 5 schematisch dargestellt. Gezeigt wird das Signal 204 beispielhaft bei einer Ausführungsform mit Insufflation 201 und Exsufflation 203, kann aber gleichermaßen auch auf Ausführungsformen übertragen werden, bei denen auf die Insufflation 201 eine Exspiration 203a folgt, ohne dass ein Unterdruck bereitgestellt wird. Nach einem Anstieg erreicht der Druck während der Insufflation 201 ein Plateauniveau, auf dem der Druck für die Insufflation 201 im Wesentlichen konstant gehalten wird und der Patient 109 mit einem Überdruck beatmet wird. Zu einer einstellbaren oder festgelegten Zeit vor dem Umschalten 202 werden dem Patient 109 durch das Signal 204 sich ändernde Atemgasparameter, hier beispielhaft das Umschalten 202 von Insufflation 201 auf Exsufflation 203, signalisiert. Sich ändernde Atemgasparameter sind dabei zumindest der Druck und/oder der Fluss, welche sich aufgrund des Umschaltens 202 ändern. Das Signal 204 wird beispielhaft für eine einstellbare oder festgelegte Zeitdauer generiert. Diese Zeitdauer kann beispielsweise zwischen 0,1 Sekunden und 2 Sekunden, bevorzugt zwischen 0,5 Sekunden und 1,5 Sekunden betragen. In manchen Ausführungsformen beträgt die Zeitdauer des Signals 204 bevorzugt zwischen 0,8 Sekunden und 1,2 Sekunden. Die Zeit vor dem Umschalten, bei der die Erzeugung des Signals 204 beginnt, liegt beispielhaft zwischen 0,1 Sekunde und 2 Sekunden, bevorzugt zwischen 0,5 Sekunden und 1,5 Sekunden, mehr bevorzugt zwischen 0,8 Sekunden und 1,2 Sekunden.

In der in Figur 5 gezeigten Ausführungsform besteht das Signal 204 aus einer Oszillation 205 des Druckes. Die Oszillation 205 kann beispielsweise durch ein periodisches Erhöhen und Verringern der Drehzahl eines Gebläses 101 und/oder durch das Schalten eines Oszillationsventil 110 erzeugt werden. In manchen Ausführungsformen kann auch ein Ventil 107, welches die Saugseite des Gebläses 101 umschaltet, eine Oszillation 205 des Druckes und somit ein pneumatisches Signal 204 erzeugen.

Die Amplitude der Oszillation 205 liegt bei 0,2 hPa bis 50 hPa, bevorzugt bei 0,5 hPa bis 30 hPa. In manchen Ausführungsformen liegt die Amplitude bevorzugt bei 1 hPa bis 20 hPa. Die Amplitude bezieht sich dabei auf den Abstand der Peaks zueinander.

Die Frequenz der Oszillation 205 liegt zwischen 1 Hz und 40 Hz, bevorzugt zwischen 2 Hz und 30 Hz. In manchen Ausführungsformen beträgt die Frequenz der Oszillation 205 bevorzugt zwischen 3 Hz und 15 Hz.

In manchen Ausführungsformen kann vorgesehen sein, dass die Frequenz und/oder Amplitude der Oszillation 205 während der Generierung des Signals sich ansteigend ändern und/oder abfallend ändern. Es kann alternativ oder ergänzend auch vorgesehen sein, dass sich die Frequenz und/oder Amplitude dynamisch ändern. In manchen Ausführungsformen kann vorgesehen sein, dass sich die Amplitude und/oder Frequenz zunächst erhöht und dann wieder abfällt. Beispielsweise können Amplitude und/oder Frequenz selbst oszillieren, also periodisch zu und abnehmen. In manchen Ausführungsformen kann vorgesehen sein, dass die Frequenz und/oder Amplitude der Oszillation 205 zum Zeitpunkt des Umschaltens 202 hin zunehmen und ihr Maximum unmittelbar vorm Umschalten 202 erreichen.

Es kann beispielhaft vorgesehen sein, dass die Zeit vor dem Umschalten 202, die Zeitdauer des Signals 204, die Frequenz und/oder Amplitude der Oszillation 205 über die Benutzerschnittstelle 104 einstellbar sind. Ergänzend oder alternativ kann vorgesehen sein, dass die Zeit vor dem Umschalten 202, die Zeitdauer des Signals 204, die Frequenz und/oder Amplitude der Oszillation 205 festgelegt sind und/oder aus festgelegten Werten ausgewählt werden können.

In manchen Ausführungsformen kann vorgesehen sein, dass die als Signal 204 verwendete Oszillation 205 auch zu Messzwecken genutzt wird, beispielsweise um über Atemgassignale auf verschiedene Atmungsparameter, z.B. dem Füllzustand der Lunge des Patienten 109 und/oder Sekretablagerungen, zu schließen.

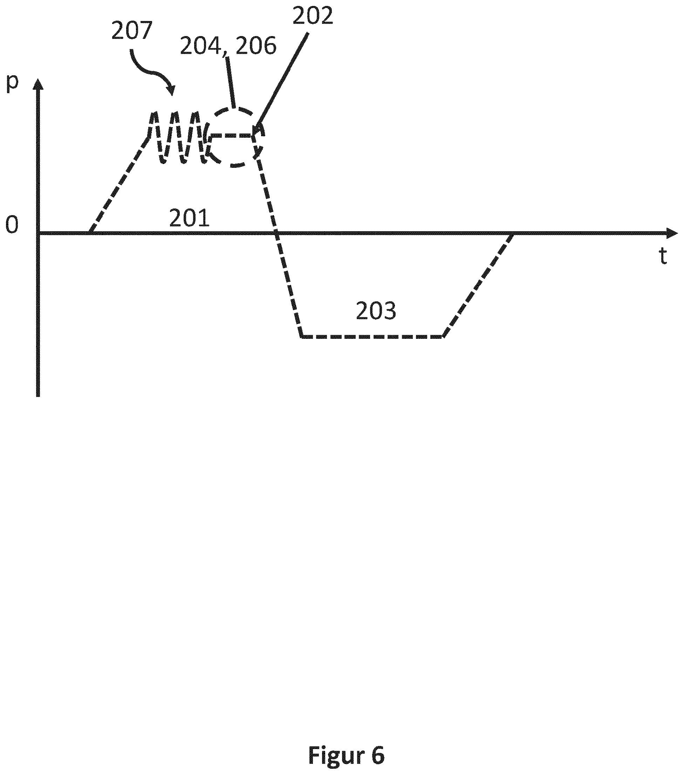

Eine weitere beispielhafte Ausführungsform des Signals 204 zur Signalisierung sich ändernder Atemparameter, insbesondere des Umschaltens 202 von Insufflation 201 auf Exsufflation 203 bzw. Exspiration 203a (hier nicht dargestellt), ist in Figur 6 schematisch dargestellt.

Beispielhaft ist die Insufflation 201 mit einer Oszillation 207 überlagert. Das Signal 204 besteht in diesem Fall in einem Wegschalten 206 der Oszillation 207. Zu einer festgelegten und/oder einstellbaren Zeit vor dem Umschalten 202 von Insufflation 201 auf Exsufflation 203 wird die Oszillation 207 weggeschaltet, um den Patienten 109 zu signalisieren, dass nach einer Zeitdauer das Umschalten 202 von Insufflation 201 auf Exsufflation 203 erfolgt.

Die Zeitdauer kann beispielsweise zwischen 0,1 Sekunden und 2 Sekunden, bevorzugt zwischen 0,5 Sekunden und 1,5 Sekunden betragen. In manchen Ausführungsformen beträgt die Zeitdauer des Signals 204 bevorzugt zwischen 0,8 Sekunden und 1,2 Sekunden. Die Zeit vor dem Umschalten, bei der die Erzeugung des Signals 204 beginnt, liegt beispielhaft zwischen 0,1 Sekunde und 2 Sekunden, bevorzugt zwischen 0,5 Sekunden und 1,5 Sekunden, mehr bevorzugt zwischen 0,8 Sekunden und 1,2 Sekunden. In manchen Ausführungsformen entspricht die Zeitdauer der Zeit vor dem Umschalten.

In manchen Ausführungsformen kann vorgesehen sein, dass die Insufflation 201 für eine festgelegte und/oder einstellbare Zeit erfolgt. Alternativ oder ergänzend kann vorgesehen sein, dass die Zeit der Insufflation 201 dynamisch angepasst wird, beispielsweise anhand von durch die Sensoren 105 aufgenommenen Werten. Beispielsweise kann ein Füllgrad der Lunge des Patienten 109 bestimmt werden. Ist ein bestimmter Füllgrad erreicht, kann vorgesehen sein, dass das Umschalten 202 signalisiert wird. In manchen Ausführungsformen kann die Oszillation 207 dazu genutzt werden, um verschiedene Atmungsparameter des Patienten 109 zu ermitteln, welche beispielsweise daraufhin ausgewertet werden, ob ein Umschalten 202 erfolgen sollte.

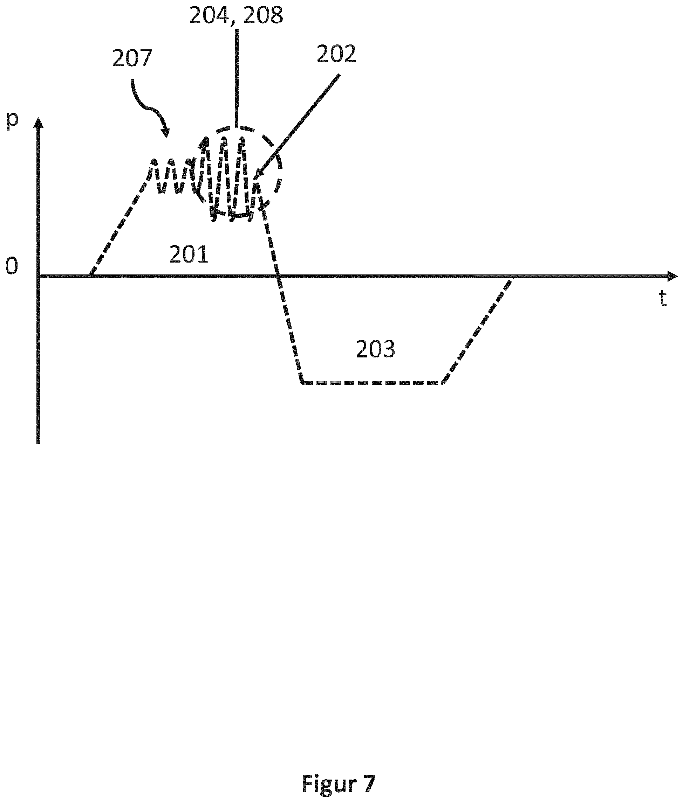

In manchen Ausführungsformen kann bei einer Oszillation 207 während der Insufflation 201 auch eine Modulation 208 der Oszillation vorgesehen sein, um sich ändernde Atemgasparameter, wie etwa das Umschalten 202 von Insufflation 201 auf Exsufflation 203, zu signalisieren. Eine solche Ausführungsform ist beispielhaft in Figur 7 dargestellt. Zu einer Zeit vor dem Umschalten 202 wird dem Patienten 109 über eine Modulation 208 der Oszillation ein pneumatisches Signal 204 generiert, welches die anstehenden sich ändernden Atemgasparameter signalisiert. Beispielhaft wird die Amplitude und/oder Frequenz der Oszillation erhöht oder verringert.

Die Amplitude der modulierten Oszillation 208 liegt bei 0,2 hPa bis 50 hPa, bevorzugt bei 0,5 hPa bis 30 hPa. In manchen Ausführungsformen liegt die Amplitude bevorzugt bei 1 hPa bis 20 hPa. Die Amplitude bezieht sich dabei auf den Abstand der Peaks zueinander.

Die Frequenz der modulierten Oszillation 208 liegt zwischen 1 Hz und 40 Hz, bevorzugt zwischen 2 Hz und 30 Hz. In manchen Ausführungsformen beträgt die Frequenz der Oszillation 205 bevorzugt zwischen 3 Hz und 15 Hz.

Die Zeitdauer des Signals 204 kann beispielsweise zwischen 0,1 Sekunden und 2 Sekunden, bevorzugt zwischen 0,5 Sekunden und 1,5 Sekunden betragen. In manchen Ausführungsformen beträgt die Zeitdauer des Signals 204 bevorzugt zwischen 0,8 Sekunden und 1,2 Sekunden. Die Zeit vor dem Umschalten, bei der die Erzeugung des Signals 204 beginnt, liegt beispielhaft zwischen 0,1 Sekunde und 2 Sekunden, bevorzugt zwischen 0,5 Sekunden und 1,5 Sekunden, mehr bevorzugt zwischen 0,8 Sekunden und 1,2 Sekunden. In manchen Ausführungsformen entspricht die Zeitdauer der Zeit vor dem Umschalten.

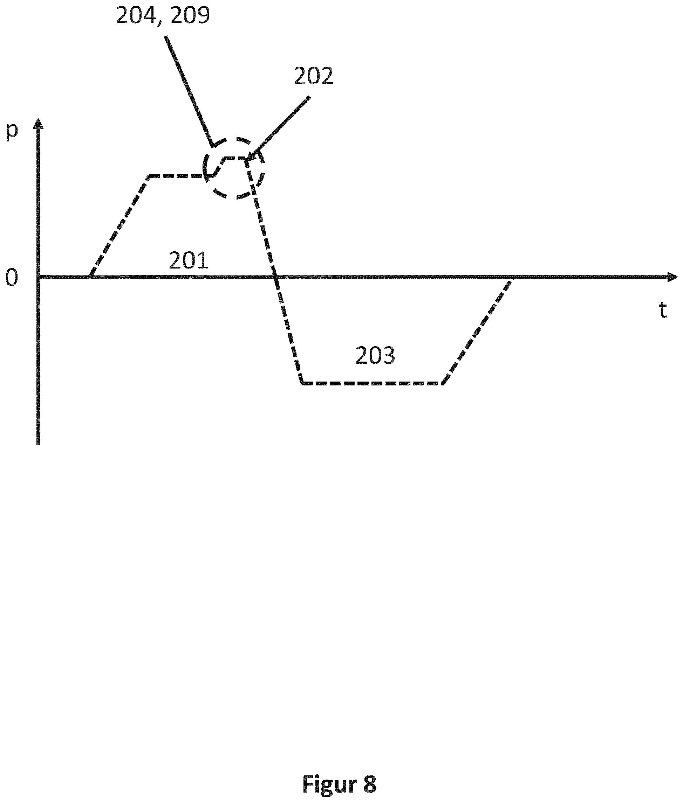

In manchen Ausführungsformen besteht das Signal 204 in einem temporären Anheben 209 des Druckes, wie in Figur 8 beispielhaft dargestellt. Dabei wird zu einer Zeit vor dem Umschalten 202 der Plateaudruck der Insufflation 201 weiter angehoben, um einen dann maximalen Druck bereitzustellen. Dieser angehobene Druck 209 bleibt beispielhaft für eine Zeitdauer bestehen. Das Umschalten von Insufflation 201 zu Exsufflation 203 erfolgt dann von dem angehobenen Druck 209 des Signals 204 ohne auf das Plateauniveau zurückzukehren. In manchen Ausführungsformen kann alternativ oder ergänzend vorgesehen sein, dass der angehobene Druck 209 nicht bis zum Umschalten 202 beibehalten wird, sondern zunächst wieder auf Plateauniveau absinkt und nach einer weiteren Zeitdauer erst das Umschalten 202 auf die Exsufflation 203 erfolgt. Alternativ oder ergänzend kann vorgesehen sein, dass der Druck stetig bis zum Umschalten zunimmt und das Maximum unmittelbar vor dem Umschalten 202 erreicht.

Die Zeitdauer des Signals 204 kann beispielsweise zwischen 0,1 Sekunden und 2 Sekunden, bevorzugt zwischen 0,5 Sekunden und 1,5 Sekunden betragen. In manchen Ausführungsformen beträgt die Zeitdauer des Signals 204 bevorzugt zwischen 0,8 Sekunden und 1,2 Sekunden. Die Zeit vor dem Umschalten, bei der die Erzeugung des Signals 204 beginnt, liegt beispielhaft zwischen 0,1 Sekunde und 2 Sekunden, bevorzugt zwischen 0,5 Sekunden und 1,5 Sekunden, mehr bevorzugt zwischen 0,8 Sekunden und 1,2 Sekunden. In manchen Ausführungsformen entspricht die Zeitdauer der Zeit vor dem Umschalten.

Zur Generierung des Signals 204 kann der Druck beispielhaft in einem Bereich von 0,2 hPa bis 8 hPa temporär angehoben werden. Bevorzugt wird der Druck in einem Bereich von 0,5 hPa bis 5 hPa temporär angehoben. Das Anheben 209 des Drucks kann beispielhaft über eine erhöhte Drehzahl des Gebläses 101 erzeugt werden.

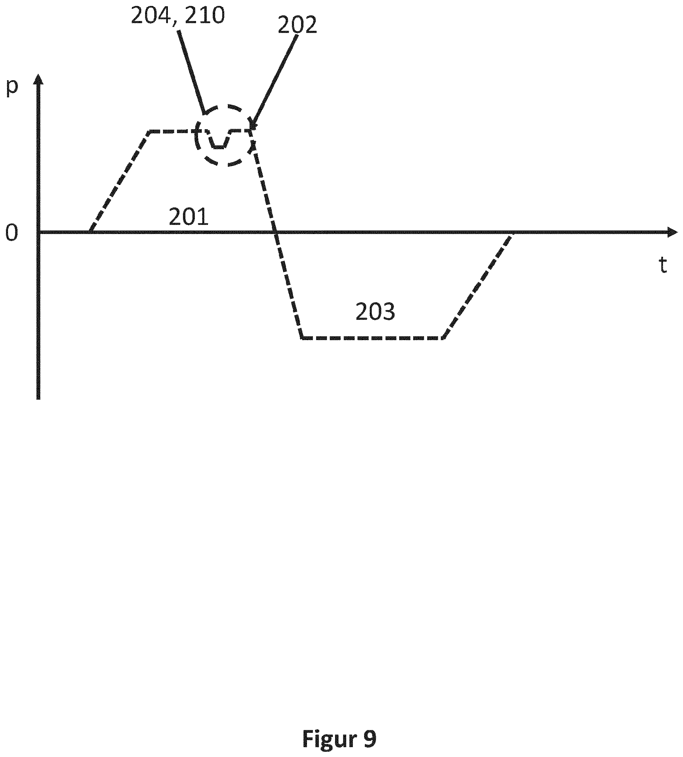

In manchen Ausführungsformen besteht das Signal 204 in einem temporären Absenken 210 des Druckes, wie in Figur 9 beispielhaft dargestellt. Dabei wird zu einer Zeit vor dem Umschalten 202 der Plateaudruck der Insufflation 201 zumindest zeitweise abgesenkt. Dieser abgesenkte Druck 210 bleibt beispielhaft für eine Zeitdauer bestehen. Das Umschalten von Insufflation 201 zu Exsufflation 203 erfolgt dann von dem abgesenkten Druck 210 des Signals 204 ohne auf das Plateauniveau zurückzukehren. In manchen Ausführungsformen kann alternativ oder ergänzend vorgesehen sein, dass der abgesenkte Druck 210 nicht bis zum Umschalten 202 beibehalten wird, sondern zunächst wieder auf Plateauniveau angehoben wird und nach einer weiteren Zeitdauer erst das Umschalten 202 auf die Exsufflation 203 erfolgt. Alternativ oder ergänzend kann vorgesehen sein, dass der Druck stetig bis zum Umschalten abnimmt und von dort direkt in die Exsufflation 203 umgeschaltet wird.

Die Zeitdauer des Signals 204 kann beispielsweise zwischen 0,1 Sekunden und 2 Sekunden, bevorzugt zwischen 0,5 Sekunden und 1,5 Sekunden betragen. In manchen Ausführungsformen beträgt die Zeitdauer des Signals 204 bevorzugt zwischen 0,8 Sekunden und 1,2 Sekunden. Die Zeit vor dem Umschalten, bei der die Erzeugung des Signals 204 beginnt, liegt beispielhaft zwischen 0,1 Sekunde und 2 Sekunden, bevorzugt zwischen 0,5 Sekunden und 1,5 Sekunden, mehr bevorzugt zwischen 0,8 Sekunden und 1,2 Sekunden. In manchen Ausführungsformen entspricht die Zeitdauer der Zeit vor dem Umschalten.

Zur Generierung des Signals 204 kann der Druck beispielhaft in einem Bereich von 0,2 hPa bis 8 hPa temporär abgesenkt werden. Bevorzugt wird der Druck in einem Bereich von 0,5 hPa bis 5 hPa temporär abgesenkt. Das Absenken 210 des Drucks kann beispielhaft über eine verringerte Drehzahl des Gebläses 101 erzeugt werden.

## Patentansprüche

1. Vorrichtung (100) zur Atmungstherapie eines Patienten mit einer Atemgasquelle (101) zur Vorgabe unterschiedlicher Atemgasparameter, mit zumindest einer Steuereinheit (103) und mit einer Signaleinheit (106) zur Ausgabe von zumindest einem Signal, wobei das Signal der Signalisierung sich ändernder Atemgasparameter dient und für den Patienten sensorisch wahrnehmbar ist wobei das Signal eine aufmodulierte Schwingung/Oszillation mit einer festen oder sich ändernden Frequenz und/oder Amplitude ist **dadurch gekennzeichnet, dass** die Signaleinheit zur Erzeugung von zumindest einem pneumatischen Signal ausgebildet ist und wobei die Vorrichtung eingerichtet ist, den Atemgasparameter beim Umschalten von der Insufflation in die Hustenphase zu verändern und wobei die Vorrichtung dazu eingerichtet ist, eine Insufflation mit überlagerter Oszillation durchzuführen, wobei das Signal eine aufmodulierte Oszillation ist, die vor der Umschaltung in die Hustenphase verändert wird.

2. Vorrichtung (100) nach zumindest einem der vorhergehenden Ansprüche, wobei die Signaleinheit (106) von der Steuereinheit (103) gesteuert wird.

3. Vorrichtung (100) nach zumindest einem der vorhergehenden Ansprüche, wobei der sich ändernde Atemgasparameter der durch die Vorrichtung bereitgestellte Atemgasdruck ist.

4. Vorrichtung (100) nach Anspruch 1, wobei der sich ändernde Atemgasparameter ein positiver Atemgasdruck ist, der im Anschluss an die Insufflationsphase für die Hustenphase abgebaut wird oder auf Unterdruck umgeschaltet wird.

5. Vorrichtung (100) nach zumindest einem der vorhergehenden Ansprüche 1 oder 4, wobei die Vorrichtung eingerichtet ist eine Insufflation ohne Oszillation durchzuführen, wobei das Signal eine spezifische Modulation des Atemgases hinsichtlich Druck und/oder Fluss und/oder Volumen ist.

6. Vorrichtung (100) nach Anspruch 1, wobei die Schwingung eine Frequenz zwischen einem und 40 Hz aufweist, bevorzugt eine Frequenz zwischen 2 und 30 Hz hat und dass die Amplitude der Schwingung bei 0,2 bis 50 hPa liegt, bevorzugt bei 0,5 bis 30 hPa liegt.

7. Vorrichtung (100) nach zumindest einem der vorhergehenden Ansprüche, wobei die Vorrichtung zudem einen Generator zur Erzeugung eines Detektorsignals und einen Sensor (105) zur Ermittlung einer Veränderung des Detektorsignals aufweist, wobei die die Vorrichtung eingrichtet ist, anhand des Detektorsignals, Veränderungen der Durchgängigkeit der Atemwege und/oder eine zumindest fortgeschrittene oder vollständige Füllung der Lunge zu detektieren und der Sensor diese Veränderung des Detektorsignals ermittelt.

8. Vorrichtung (100) nach Anspruch 1, wobei eine automatische Umschaltung in die Hustenphase erfolgt, wenn basierend auf dem Detektorsignal eine Veränderung der Durchgängigkeit der Atemwege oder eine zumindest fortgeschrittene oder vollständige Füllung der Lunge ermittelt wird.

9. Vorrichtung (100) nach zumindest einem der vorhergehenden Ansprüche 1 oder 8, wobei die automatische Umschaltung auf einem oszillatorischen Druck-, Fluss- und/oder Volumensignal basiert.

10. Vorrichtung (100) nach zumindest einem der vorhergehenden Ansprüche 1, 8 oder 9, wobei die Umschaltung auch vor Ablauf einer hinterlegten oder eingestellten Zeitdauer stattfindet, wenn der Glottisschluss messtechnisch durch ein Detektorsignal festgestellt ist.

11. Vorrichtung (100) nach zumindest einem der vorhergehenden Ansprüche 1, 4 oder 5, wobei die Oszillation während der Insufflation stattfindet, wobei die Oszillation gleichzeitig als Basis für das Detektorsignal verwendet wird und eine Umschaltung dann erfolgt, wenn basierend auf dem Detektorsignal auf einen Verschluss der Glottis und/oder auf eine zumindest weitgehend vollständig gefüllte Lunge vor Ablauf der eingestellten Insufflationszeit geschlossen werden kann.

12. System zur Atmungstherapie eines Patienten umfassend eine Vorrichtung (100) nach zumindest einem der vorhergehenden Ansprüche, wobei das System zudem ein Patienteninterface und einen Atemgasschlauch umfasst.

## Claims

1. An apparatus (100) for respiratory therapy of a patient with a respiratory gas source (101) for specifying different respiratory gas parameters, with at least one control unit (103) and with a signal unit (106) for outputting at least one signal, wherein the signal serves to signal changing respiratory gas parameters and is perceptible to the patient through the senses, wherein the signal is a modulated vibration/oscillation with a fixed or variable frequency and/or amplitude, **characterized in that**
the signal unit is designed to generate at least one pneumatic signal and wherein the apparatus is configured to change the respiratory gas parameter when switching from insufflation to the cough phase and wherein the apparatus is configured to perform insufflation with a superimposed oscillation, wherein the signal is a modulated oscillation that is changed before the switch to the cough phase.

2. The apparatus (100) according to at least one of the preceding claims, wherein the signal unit (106) is controlled by the control unit (103).

3. The apparatus (100) according to at least one of the preceding claims, wherein the variable respiratory gas parameter is the respiratory gas pressure provided by the apparatus.

4. The apparatus (100) according to claim 1, wherein the variable respiratory gas parameter is a positive respiratory gas pressure which is reduced or is switched to negative pressure for the cough phase at the conclusion of the insufflation phase.

5. The apparatus (100) according to at least one of the preceding claims 1 or 4, wherein the apparatus is configured to perform insufflation without oscillation, wherein the signal is a specific modulation of the respiratory gas with regard to pressure and/or flow and/or volume.

6. The apparatus (100) according to claim 1, wherein the vibration has a frequency between one and 40 Hz, preferably a frequency between 2 and 30 Hz, and that the amplitude of the vibration is 0.2 to 50 hPa, preferably 0.5 to 30 hPa.

7. The apparatus (100) according to at least one of the preceding claims, wherein the apparatus also has a generator for generating a detector signal and a sensor (105) for determining a change in the detector signal, wherein the apparatus is configured to detect, using the detector signal, changes in the patency of the airways and/or an at least advanced or complete filling of the lung and the sensor determines this change in the detector signal.

8. The apparatus according to claim 1, wherein an automatic switch to the cough phase occurs when, based on the detector signal, a change in the patency of the airways or an at least advanced or complete filling of the lung is determined.

9. The apparatus (100) according to at least one of the preceding claims 1 or 8, wherein the automatic switch is based on an oscillatory pressure, flow, and/or volume signal.

10. The apparatus (100) according to at least one of the preceding claims 1, 8, or 9, wherein the switch also takes places before a saved or set time duration has elapsed when glottal closure is ascertained by measurement by means of a detector signal.

11. The apparatus (100) according to at least one of the preceding claims 1, 4, or 5, wherein the oscillation takes place during insufflation, wherein the oscillation is used simultaneously as the basis for the detector signal and a switch occurs when, based on the detector signal, a closure of the glottis and/or an at least largely completely filled lung can be inferred before the set insufflation time has elapsed.

12. A system for respiratory therapy of a patient comprising an apparatus (100) according to at least one of the preceding claims, wherein the system also comprises a patient interface and a respiratory gas hose.

## Revendications

1. Appareil (100) de thérapie respiratoire d'un patient, comprenant une source de gaz respiratoire (101) pour la préconfiguration de différents paramètres de gaz respiratoire, au moins une unité de commande (103) et une unité de signal (106) destinée à émettre au moins un signal, dans lequel le signal sert à signaler des paramètres de gaz respiratoire changeants et est perceptible sensoriellement pour le patient, dans lequel le signal est une vibration/oscillation modulée avec une fréquence et/ou une amplitude fixe ou changeante, **caractérisé en ce que** l'unité de signal est conçue pour produire au moins un signal pneumatique et dans lequel l'appareil est configuré pour modifier le paramètre de gaz respiratoire lors de la commutation de l'insufflation à la phase de toux et dans lequel l'appareil est configuré pour effectuer une insufflation avec une oscillation superposée, dans lequel le signal est une oscillation modulée, laquelle est modifiée avant la commutation à la phase de toux.

2. Appareil (100) selon l'une au moins des revendications précédentes, dans lequel l'unité de signal (106) est commandée par l'unité de commande (103).

3. Appareil (100) selon l'une au moins des revendications précédentes, dans lequel le paramètre de gaz respiratoire changeant est la pression de gaz respiratoire fournie par l'appareil.

4. Appareil (100) selon la revendication 1, dans lequel le paramètre de gaz respiratoire changeant est une pression de gaz respiratoire positive évacuée après la phase d'insufflation pour la phase de toux ou commutée sur une pression négative.

5. Appareil (100) selon l'une au moins des revendications précédentes 1 ou 4, dans lequel l'appareil est configuré pour effectuer une insufflation sans oscillation, dans lequel le signal est une modulation spécifique du gaz respiratoire quant à la pression et/ou au flux et/ou au volume.

6. Appareil (100) selon la revendication 1, dans lequel la vibration présente une fréquence entre un et 40 Hz, de préférence une fréquence entre 2 et 30 Hz, et en ce que l'amplitude de la vibration se situe entre 0,2 et 50 hPa, de préférence entre 0,5 et 30 hPa.

7. Appareil (100) selon l'une au moins des revendications précédentes, dans lequel l'appareil présente en outre un générateur destiné à produire un signal de détecteur et un capteur (105) destiné à déterminer une modification du signal de détecteur, dans lequel l'appareil est configuré pour détecter, à l'aide du signal de détecteur, des modifications de la perméabilité des voies respiratoires et/ou un remplissage au moins avancé ou complet des poumons et le capteur détermine cette modification du signal de détecteur.

8. Appareil selon la revendication 1, dans lequel une commutation automatique à la phase de toux a lieu lorsqu'une modification de la perméabilité des voies respiratoires ou un remplissage au moins avancé ou complet des poumons est déterminé sur la base du signal de détecteur.

9. Appareil (100) selon l'une au moins des revendications précédentes 1 ou 8, dans lequel la commutation automatique est basée sur un signal oscillatoire de pression, de flux et/ou de volume.

10. Appareil (100) selon l'une au moins des revendications précédentes 1, 8 ou 9, dans lequel la commutation a lieu également avant l'écoulement d'une période enregistrée ou réglée, lorsqu'un coup de glotte est constaté à l'aide d'une technique de mesure par un signal de détecteur.

11. Appareil (100) selon l'une au moins des revendications précédentes 1, 4 ou 5, dans lequel l'oscillation se produit pendant l'insufflation, dans lequel l'oscillation est utilisée simultanément comme base pour le signal de détecteur et une commutation a lieu lorsqu'il est possible de conclure, sur la base du signal de détecteur, à un coup de glotte et/ou à un remplissage au moins quasiment complet des poumons avant l'écoulement du temps d'insufflation réglé.

12. Système de thérapie respiratoire d'un patient comportant un appareil (100) selon l'une au moins des revendications précédentes, dans lequel le système comporte en outre une interface patient et un tube de gaz respiratoire.
